(19) Europäisches Patentamt
European Patent Office
Office européen des brevets

(11) Veröffentlichungsnummer: **0 154 842**
**A2**

(12) **EUROPÄISCHE PATENTANMELDUNG**

(21) Anmeldenummer: **85101744.2**

(22) Anmeldetag: **16.02.85**

(51) Int. Cl.⁴: **A 61 K 31/47**
// C07D217/02, C07D217/04

(30) Priorität: **03.03.84 DE 3407955**

(43) Veröffentlichungstag der Anmeldung: **18.09.85**
**Patentblatt 85/38**

(84) Benannte Vertragsstaaten: **BE CH DE FR GB IT LI LU NL SE**

(71) Anmelder: **Dr. Karl Thomae GmbH, Postfach 1755, D-7950 Biberach (Riss) (DE)**

(72) Erfinder: **Nicki, Josef, Dr. Dipl.-Chem., Silcherstrasse 8, D-7950 Biberach 1 (DE)**
Erfinder: **Müller, Erich, Dr. Dipl.-Chem., Talfeldstrasse 34, D-7950 Biberach 1 (DE)**
Erfinder: **Narr, Berthold, Dr. Dipl.-Chem., Obere Au 5,, D-7950 Biberach 1 (DE)**
Erfinder: **Ballhause, Helmut, Fohrenweg 6, D-7950 Biberach 1 (DE)**
Erfinder: **Haarmann, Walter, Dr., Schlierholzweg 27, D-7950 Biberach 1 (DE)**

(54) **Arzneimittel, enthaltend quartäre 3,4-Dihydro-isochinolinium salze.**

(57) Gegenstand der vorliegenden Erfindung sind neue Arzneimittel mit antithrombotischen Wirkungen, enthaltend eine Verbindung der obigen allgemeinen Formel

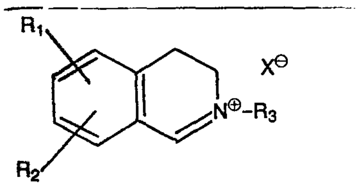

(I)

in der $R_1$ und $R_2$, die gleich oder verschieden sein können, Wasserstoffatome, Alkyl- oder Alkoxygruppen,
$R_3$ eine Aralkylgruppe, deren Phenylkern durch Halogenatome, Alkyl- oder Alkoxygruppen mono-, di- oder trisubstituiert sein kann, wobei die Substituenten des Phenylkerns gleich oder verschieden sein können, und
X das Anion einer physiologisch verträglichen anorganischen oder organischen Säure bedeuten, deren Verwendung und Herstellung.

EP 0 154 842 A2

0154842
Case 5/900
Dr.Fl/pf

DR. KARL THOMAE GMBH
D-7950 BIBERACH

# Arzneimittel, enthaltend quartäre 3,4-Dihydro-isochinoliniumsalze

In der Literatur werden bereits quartäre 3,4-Dihydro-isochinoliniumsalze beschrieben (siehe beispielsweise Chim. Ther. 6, 358-366 und 462-468 (1971), J. C. S. Chem. Comm. 19, 740-741 (1973), Can. J. Chem. 58, 2770-2779 (1980) und J. Org. Chem. 45, 3176-3181 (1980)). Diese Verbindungen stellen Zwischenprodukte u.a. zur Herstellung von 1,2,3,4-Tetrahydro-isochinolinen mit wertvollen pharmakologischen Eigenschaften dar.

Es wurde nun gefunden, daß die 3,4-Dihydro-isochinoliniumsalze der allgemeinen Formel

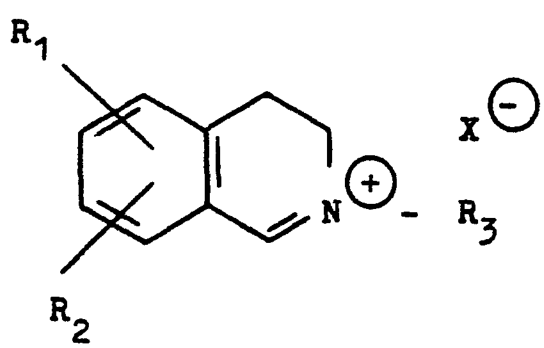

(I)

in der

$R_1$ und $R_2$, die gleich oder verschieden sein können, Wasserstoffatome, Alkyl- oder Alkoxygruppen mit jeweils 1 bis 3 Kohlenstoffatomen im Alkylteil,

$R_3$ eine Aralkylgruppe mit 7 bis 9 Kohlenstoffatomen, deren Phenylkern durch Halogenatome, Alkyl- oder Alkoxygruppen mit jeweils 1 bis 3 Kohlenstoffatomen im Alkylteil mono-, di- oder trisubstituiert sein kann, wobei die Substituenten des Phenylkerns gleich oder verschieden sein können, und

X das Anion einer physiologisch verträglichen anorganischen oder organischen Säure bedeuten, wertvolle pharmakologische Eigenschaften aufweisen, insbesondere eine antithrombotische Wirkung.

Gegenstand der vorliegenden Erfindung sind somit neue Arzneimittel mit antithrombotischen Wirkungen, enthaltend eine Verbindung der obigen allgemeinen Formel I, deren Verwendung und Herstellung sowie die neuen Verbindungen.

Für die bei der Definition der Reste $R_1$ bis $R_3$ und X eingangs erwähnten Bedeutungen kommen beispielsweise

für $R_1$ und $R_2$, die gleich oder verschieden sein können, jeweils die Bedeutung des Wasserstoffatoms, der Methyl-, Äthyl-, n-Propyl-, Isopropyl-, Methoxy-, Äthoxy-, n-Propoxy- oder Isopropoxygruppe,

für $R_3$ die der Benzyl-, 1-Phenyläthyl-, 2-Phenyläthyl-, 1-Phenyl-n-propyl-, 2-Phenyl-n-propyl-, 3-Phenyl-n-propyl-, 1-Methyl-2-phenyl-äthyl-, Fluorbenzyl-, Chlorbenzyl-, Brombenzyl-, Difluorbenzyl-, Dichlorbenzyl-, Dibrombenzyl-, Methylbenzyl-, Äthylbenzyl-, Isopropylbenzyl-, Dimethylbenzyl-, Äthyl-methylbenzyl-, Trimethylbenzyl-, Methoxybenzyl-, Dimethoxybenzyl-, Trimethoxybenzyl-, Äthoxybenzyl-, Diäthoxybenzyl-, Chlor-methylbenzyl-, Chlor-methoxybenzyl-, Brom-chlorbenzyl-, Brom-methylbenzyl-, Brom-methoxybenzyl-, 2-(Chlorphenyl)-äthyl-, 2-(Bromphenyl)-äthyl-, 2-(Methylphenyl)-äthyl-, 2-(Methoxyphenyl)-äthyl-, 3-(Chlorphenyl)-n-propyl-, 3-(Bromphenyl)-n-propyl-, 3-(Methylphenyl)-n-propyl- oder 3-(Methoxyphenyl)-n-propylgruppe und

für X die des Anions der Salzsäure, Bromwasserstoffsäure, Schwefelsäure, Phosphorsäure, Essigsäure oder p-Toluolsulfonsäure in Betracht.

Bevorzugte Verbindungen der obigen allgemeinen Formel I sind diejenigen, in denen $R_1$ und $R_2$, die gleich oder verschieden sein können, Wasserstoffatome, Methyl- oder Methoxygruppen und

$R_3$ eine Benzyl-, Fluorbenzyl-, Chlorbenzyl-, Brombenzyl-, Methoxybenzyl- oder Trimethoxybenzylgruppe bedeuten,

insbesondere jedoch diejenigen Verbindungen in der $R_1$ und $R_2$ je eine Methoxygruppe, $R_3$ eine 2-Chlorbenzyl-, 2-Methoxybenzyl- oder 3,4,5-Trimethoxybenzylgruppe und

X das Anion einer physiologisch verträglichen Säure bedeuten.

Die Verbindungen der obigen allgemeinen Formel erhält man beispielsweise nach folgenden Verfahren:

a.) Umsetzung eines 3,4-Dihydro-isochinolins der allgemeinen Formel

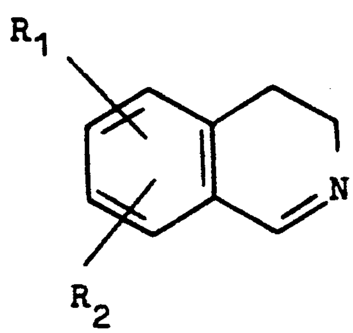

(II)

in der

$R_1$ und $R_2$ wie eingangs definiert sind, mit einer Verbindung der allgemeinen Formel

$$R_3 - Y \qquad \text{(III)}$$

in der

$R_3$ wie eingangs definiert ist und

Y einen nukleophil austauschbaren Rest wie ein Halogenatom oder einen Sulfonsäurerest, z.B. ein Chlor-, Brom- oder Jodatom, eine Methansulfonyloxy- oder p-Toluolsulfonyloxygruppe, bedeuten.

Die Umsetzung wird zweckmäßigerweise in einem Lösungsmittel oder Lösungsmittelgemisch wie Diäthyläther, Tetrahydrofuran, Dioxan, Toluol, Essigsäureäthylester, Methylenchlorid, Äthylenchlorid, Chloroform, Äthanol, Isopropanol, Acetonitril oder Tetrahydrofuran/Toluol bei Temperaturen zwischen 0 und 150°C, vorzugsweise jedoch bei Temperaturen zwischen 10 und 60°C, durchgeführt. Die Umsetzung kann jedoch auch ohne Lösungsmittel durchgeführt werden.

b.) Cyclisierung einer gegebenenfalls im Reaktionsgemisch gebildeten N-Formyl-Verbindung der allgemeinen Formel

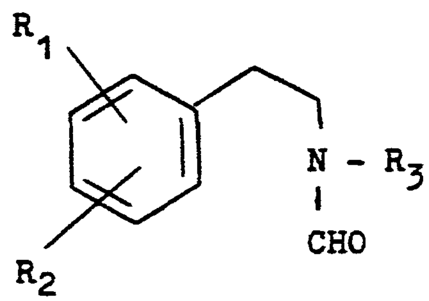

(IV)

in der

$R_1$ bis $R_3$ wie eingangs definiert sind, in Gegenwart eines sauren Kondensationsmittels.

Die Umsetzung wird in Gegenwart eines sauren Kondensationsmittel wie Polyphosphorsäure, konzentrierte Schwefelsäure oder Phosphoroxychlorid gegebenenfalls in einem Lösungsmittel wie Toluol, Chlorbenzol, Xylol oder Dichlorbenzol bei erhöhten Temperaturen, z.B. bei Temperaturen zwischen 50°C und der Siedetemperatur des Reaktionsgemisches, durchgeführt. Die Umsetzung kann jedoch auch ohne Lösungsmittel durchgeführt werden.

Erhält man gemäß dem Verfahren a) oder b) eine Verbindung der allgemeinen Formel I, in der X ein anderes als das gewünschte Anion darstellt, so kann diese Verbindung durch Überführung in ihre Base, z.B. mittels eines Alkalihydroxids wie Natron- oder Kalilauge, und anschließendes Versetzen mit einer entsprechenden Säure in die gewünschte Verbindung übergeführt werden.

Die als Ausgangsstoffe verwendeten Verbindungen der allgemeinen Formeln II bis IV sind literaturbekannt bzw. man erhält diese ebenfalls nach literaturbekannten Verfahren. So erhält man ein 3,4-Dihydro-isochinolin der allgemeinen Formel II durch Cyclisierung eines entsprechenden N-Formyl-2-phenyläthylamins und eine Verbindung der allgemeinen Formel IV durch Formylierung eines entpsrechenden N-Aralkyl-2-phenyläthylamins.

Wie bereits eingangs erwähnt, weisen die Verbindungen der allgemeinen Formel I wertvolle pharmakologische Eigenschaften auf, nämlich neben einer Hemmwirkung auf die Aggregationsneigung von Tumorzellen insbesondere eine antithrombotische Wirkung.

Beispielsweise wurden die Verbindungen

A = 2-(2-Chlorbenzyl)-6,7-dimethoxy-3,4-dihydro-isochinolinium-hydrogensulfat,

B = 2-(2-Chlorbenzyl)-3,4-dihydro-isochinolinium-hydrogensulfat und

C = 2-(3,4,5-Trimethoxybenzyl)-6,7-dimethoxy-3,4-dihydroisochinolinium-chlorid

auf ihre biologischen Eigenschaften wie folgt untersucht:

1.) Antithrombotische Wirkung (siehe BORN und CROSS, J. Physiol. 170, 397 (1964)):

Jeweils 3 männlichen Ratten pro Behandlungsgruppe im Gewicht von etwa 500 g werden die zu untersuchenden Substanzen in 1%iger Tylose suspendiert (A und B) bzw. in Aqua dest. gelöst (C), in einem Volumen von 0,2 ml pro 100 g Tiergewicht per Schlundsonde verabreicht. Kurz vor der Blutentnahme (1 Stunde nach Substanzgabe) werden die Tiere mit Pentobarbital-Natrium (50 mg/kg i.p.) narkotisiert. Nach Eröffnung des Abdomens wird per Aortenpunktion die benötigte Blutmenge entnommen.

Das Blut wird mit Natriumcitrat (0,2 % Endkonzentration) anticoaguliert. Da die Aggregation vor und nach Substanzgabe bei Ratten nicht am selben Tiere gemessen werden kann, hat es sich als zweckmäßig erwiesen, Tierkollektive miteinander zu vergleichen, deren Plasmen gepoolt wurden. So können auch Mehrfachbestimmungen durchgeführt werden.

Die Thrombozytenaggregation wird in plättchenreichem Plasma (PRP) gemessen (Plasma-Menge pro Versuch: 1 ml). Zur PRP-Gewinnung wird das Blut für 10 Minuten bei 1000 bis 1200 Umdrehungen pro Minute (ca. 150 g) zentrifugiert. Das plättchenarme Plasma zur Eichung des Aggregometers wird durch Zentrifugieren bei 4000 U/min für 10 Minuten Dauer gewonnen.

Der Verlauf der Abnahme der optischen Dichte ("optical density") der Plättchensuspension nach Zugabe von Collagen wird photometrisch gemessen und registriert. Die Kurvenhöhe in mm, die zur Zeit der maximalen Lichtdurchlässigkeit gemessen wird, ist das Maß für die Aggregationsstärke. Für die Messungen wird ein 6-Kanal-Aggregometer verwendet.

Die Collagen-Menge (Collagen der Firma Hormonchemie, 2 bis 5 µl/ml Plasma) wird so gewählt, daß sich eine irreversibel verlaufende Reaktionskurve ergibt.

Die substanzbehandelten Tierkollektive werden mit unbehandelten Kontrollkollektiven verglichen.

| Substanz | Dosis mg/kg p.o. | Aggregationsstärke, mm Kurvenhöhe (opt.density) Kontrolle | Substanz | prozentuale Aggr.-Hemmung |
|---|---|---|---|---|
| A | 10 | 130 | 69 | 47 |
| B | 5 | 108 | 0 | 100 |
| | 10 | 110 | 0 | 100 |
| C | 5 | 113 | 10 | 89 |

2. Akute Toxizität:

Die Verbindungen A, B und C sind gut verträglich, da sie bei den höchsten applizierten Dosen (10 mg/kg p.o.) keinerlei toxische Nebenwirkungen aufweisen.

Die orientierende akute Toxizität der Substanz B an der Maus wurde nach oraler Gabe einer Dosis von 250 mg/kg an einer Gruppe von 10 Tieren geprüft. Hierbei verstarb während der Beobachtungszeit von 14 Tagen keines der Tiere.

Erfindungsgemäß eignen sich die Verbindungen der allgemeinen Formel I aufgrund ihrer pharmakologischen Eigenschaften zur Prophylaxe thromboembolischer Erkrankungen wie Coronarinfarkt, Cerebralinfarkt, sogn. transient ischaemic attacks, Amaurosis fugax sowie zur Prophylaxe der Arteriosklerose und der Metastasenbildung.

Hierzu lassen sich diese zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser/Äthanol, Wasser/Glycerin, Wasser/Sorbit, Wasser/Polyäthylenglykol, Propylenglykol, Cetylstearylalkohol, Carboxymethylcellulose oder fetthaltiger Substanzen wie Hartfett, Paraffin, Polyoxyäthylenstearat, Decyloleat, Walrat, Sorbitanmonostearat oder deren geeignete Gemische, gegebenenfalls in Kombination mit anderen Wirksubstanzen, in die üblichen galenischen Zubereitungsformen wie Dragées, Tabletten, Kapseln, Suppositorien, Ampullen, Tropfen oder Suspensionen einarbeiten. Die Tagesdosis beträgt am Erwachsenen zweckmäßigerweise 500 bis 1000 mg, vorzugsweise 600 bis 900 mg, verteilt auf 2 bis 4 Einzeldosen.

Die nachfolgenden Beispiele sollen die Erfindung näher erläutern:

Beispiel A

6,7-Dimethoxy-3,4-dihydro-isochinolin

a.) N-Formyl-2-(3,4-dimethoxyphenyl)-äthylamin

181,2 g (1 Mol) 2-(3,4-Dimethoxyphenyl)-äthylamin und 181,9 g (1,1 Mol) Chloralhydrat werden vermischt und unter Rühren auf 120°C erhitzt. Man erhitzt 20 Minuten auf 120°C und entfernt im Wasserstrahlvakuum bei 140°C Ölbadtemperatur flüchtige Bestandteile (Chloroform und Wasser). Es hinterbleibt ein Öl, das ohne weitere Reinigung weiterverarbeitet wird.

b.) 6,7-Dimethoxy-3,4-dihydroisochinolin

Das gemäß Beispiel a) erhaltene N-Formyl-2-(3,4-dimethoxyphenyl)-äthylamin wird mit 1 kg Polyphosphorsäure verrührt und auf 140°C (Ölbadtemperatur) erhitzt, wobei die Reaktionsmischung ab einer Innentemperatur von 70°C stark zu schäumen beginnt. Nach 30 Minuten wird eine Innentemperatur von ca. 140°C erreicht. Man hält anschließend noch 30 Minuten auf dieser Temperatur. Anschließend wird der Ansatz auf 1 l Wasser gegossen und unter Zugabe von Eis mit 1,8 l konz. Ammoniak alkalisch gestellt. Man extrahiert das Reaktionsprodukt mehrmals mit Essigester, wäscht die Essigesterphase mit Wasser, trocknet über Magnesiumsulfat und dampft ein.
Ausbeute: 142,5 g (75 % der Theorie) Öl.

Zur weiteren Reinigung wird die Base in 350 ml Äthanol gelöst und mit 40 ml konz. Schwefelsäure versetzt. Das ausgefallene Salz wird nach dem Abkühlen abgesaugt, mit kaltem Äthanol gewaschen und getrocknet (Umlufttrockenschrank, 60°C).
Ausbeute: 179,5 g (62 % der Theorie),
Schmelzpunkt des Hydrogensulfates: 185-187°C

Analog werden folgende Verbindungen erhalten:

3,4-Dihydroisochinolin
$Kp_{208\ Pa}$: 110°C

6-Methoxy-3,4-dihydroisochinolin
Schmelzpunkt des Hydrogensulfates: 166-168°C (aus Äthanol)

5-Methyl-3,4-dihydroisochinolin
Schmelzpunkt des Hydrogensulfates: 188-190°C (aus Äthanol)

6,7-Dimethyl-3,4-dihydroisochinolin
Schmelzpunkt des Hydrogensulfates: 183-185°C (aus Äthanol)

## Beispiel B

### 2-(2-Chlorbenzyl)-6,7-dimethoxy-3,4-dihydroisochinolinium-hydrogensulfat

173,7 g (0,6 Mol) 6,7- Dimethoxy-3,4-dihydroisochinolin-hydrogensulfat werden in 300 ml Wasser gelöst und mittels konz. Ammoniak in die freie Base übergeführt, welche 4mal mit Essigester extrahiert wird. Man wäscht mit Wasser und trocknet die Essigesterphase und dampft im Vakuum ein. Die erhaltene Base (108,3 g = 0,57 Mol) löst man in 300 ml trockenem Dioxan und gibt 100 g (0,62 Mol) 2-Chlorbenzylchlorid zu. Nach 10 Minuten schon beginnt das 6,7-Dimethoxy-3,4-dihydroisochinolinium-chlorid auszufallen. Man rührt 4 Tage bei Raumtemperatur und setzt am 3. Tag noch einmal 10 ml (12,7 g) 2-Chlorbenzylchlorid zu.
Nach 4 Tagen saugt man das Salz ab, wäscht mit Dioxan und Äther und trocknet.
Ausbeute: 177,1 g (84 % der Theorie),
Schmelzpunkt des Chlorids: 196°C (aus Äthanol/Essigsäure-äthylester)

$C_{18}H_{19}Cl_2NO_2$ (352,27)

| | | | | | | | | |
|---|---|---|---|---|---|---|---|---|
| Ber.: | C | 61,37 | H | 5,44 | N | 3,98 | Cl | 20,13 |
| Gef.: | | 61,24 | | 5,63 | | 3,88 | | 19,98 |

Zur Überführung in das Hydrogensulfat löst man das Chlorid in Wasser, stellt mit 15 n Natronlauge alkalisch, wobei ein dicker Niederschlag ausfällt. Man extrahiert diesen mit Chloroform, wäscht, trocknet und dampft ein. Der erhaltene Rückstand wird in 450 ml Isopropanol gelöst und vorsichtig mit 27,5 ml konz. Schwefelsäure versetzt. Das kristallin ausgefallene Salz wird nach dem Kühlen abgesaugt, mit kaltem Isopropanol und Äther gewaschen und getrocknet.
Ausbeute: 138,4 g (66 % der Theorie),
Schmelzpunkt: 227-229°C (aus Methanol)
$C_{18}H_{20}ClNO_6S$ (413,89)

| | | | | | | | | | | |
|---|---|---|---|---|---|---|---|---|---|---|
| Ber.: | C | 52,24 | H | 4,87 | N | 3,38 | Cl | 8,57 | S | 7,75 |
| Gef.: | | 52,51 | | 4,85 | | 3,41 | | 8,62 | | 7,93 |

Beispiel C

2-(2-Chlorbenzyl)-3,4-dihydroisochinolinium-hydrogensulfat

Hergestellt analog Beispiel B aus 3,4-Dihydroisochinolin und 2-Chlorbenzylchlorid in Acetonitril.
Ausbeute: 75 % der Theorie,
Schmelzpunkt des Hydrogensulfates: 190-192°C (aus Isopropanol).
$C_{16}H_{16}ClNO_4S$ (353,84)

| | | | | | | | | | | |
|---|---|---|---|---|---|---|---|---|---|---|
| Ber.: | C | 54,31 | H | 4,56 | N | 3,96 | Cl | 10,02 | S | 9,06 |
| Gef.: | | 54,00 | | 4,72 | | 3,96 | | 10,05 | | 9,01 |

Beispiel D

2-(2-Chlorbenzyl)-5-methyl-3,4-dihydroisochinolinium-chlorid

Hergestellt analog Beispiel B aus 5-Methyl-3,4-dihydroisochinolin und 2-Chlorbenzylchlorid in Dioxan.
Ausbeute: 58 % der Theorie,
Schmelzpunkt: 166-168°C.

Beispiel E

2-(2-Chlorbenzyl)-6,7-dimethyl-3,4-dihydroisochinolinium-chlorid

Hergestellt analog Beispiel B aus 6,7-Dimethyl-3,4-dihydro-isochinolin und 2-Chlorbenzylchlorid in Dioxan.
Ausbeute: 62 % der Theorie,
Schmelzpunkt: 127-129°C.

Beispiel F

2-(2-Chlorbenzyl)-6-methoxy-3,4-dihydroisochinolinium-chlorid

Hergestellt analog Beispiel B aus 6-Methoxy-3,4-dihydro-isochinolin und 2-Chlorbenzylchlorid in Äthylenchlorid.
Ausbeute: 95 % der Theorie,
Schmelzpunkt: 156-158°C (Zers., aus Dioxan).

Beispiel G

2-Benzyl-6,7-dimethoxy-3,4-dihydroisochinolinium-chlorid

Hergestellt analog Beispiel B aus 6,7-Dimethoxy-3,4-dihydro-isochinolin und Benzylchlorid in Dioxan.
Ausbeute: 30 % der Theorie,
Schmelzpunkt: 181-183°C (Zers.)

$C_{18}H_{20}ClNO_2$ (317,82)

| | | | | | | | | |
|---|---|---|---|---|---|---|---|---|
| Ber.: | C | 68,02 | H | 6,34 | N | 4,41 | Cl | 11,16 |
| Gef.: | | 67,81 | | 6,16 | | 4,35 | | 11,05 |

Beispiel H

2-(2-Fluorbenzyl)-6,7-dimethoxy-3,4-dihydroisochinolinium-chlorid

Hergestellt analog Beispiel B aus 6,7-Dimethoxy-3,4-dihydroisochinolin und 2-Fluorbenzylchlorid in Äthylenchlorid.
Ausbeute: 71 % der Theorie,
Schmelzpunkt: 196-198°C (Zers.)
$C_{18}H_{19}ClFNO_2$ (335,82)

| | | | | |
|---|---|---|---|---|
| Ber.: | N | 4,17 | Cl | 10,56 |
| Gef.: | | 4,05 | | 10,60 |

Beispiel I

2-(2-Methylbenzyl)-6,7-dimethoxy-3,4-dihydroisochinolinium-chlorid

Hergestellt analog Beispiel B aus 6,7-Dimethoxy-3,4-dihydroisochinolin und 2-Methyl-benzylchlorid in Dioxan.
Ausbeute: 60 % der Theorie,
Schmelzpunkt: 193-195°C (Zers., aus Äthanol-Äther).
$C_{19}H_{22}ClNO_2$ (331,85)

| | | | | |
|---|---|---|---|---|
| Ber.: | N | 4,22 | Cl | 10,67 |
| Gef.: | | 3,96 | | 10,47 |

Beispiel J

2-(4-Methylbenzyl)-6,7-dimethoxy-3,4-dihydroisochinolinium-chlorid

Hergestellt analog Beispiel B aus 6,7-Dimethoxy-3,4-dihydro-isochinolin und 4-Methylbenzylchlorid in Äthylenchlorid.
Ausbeute: 57 % der Theorie,
Schmelzpunkt: 181-183°C (Zers.).

Beispiel K

2-(4-Chlorbenzyl)-6,7-dimethoxy-3,4-dihydroisochinolinium-chlorid

Hergestellt analog Beispiel B aus 6,7-Dimethoxy-3,4-dihydro-isochinolin und 4-Chlorbenzylchlorid in Äthylenchlorid.
Ausbeute: 85 % der Theorie,
Schmelzpunkt: 182-184°C (Zers.)
$C_{18}H_{19}Cl_2NO_2$ (352,27)

| | | | | | | | | |
|---|---|---|---|---|---|---|---|---|
| Ber.: | C | 61,37 | H | 5,44 | N | 3,98 | Cl | 20,13 |
| Gef.: | | 61,76 | | 5,46 | | 3,93 | | 20.50 |

Beispiel L

2-(3,4,5-Trimethoxybenzyl)-6,7-dimethoxy-3,4-dihydro-isochinolinium-chlorid

Hergestellt analog Beispiel B aus 6,7-Dimethoxy-3,4-dihydro-

isochinolin und 3,4,5-Trimethoxybenzylchlorid in Dioxan.
Ausbeute: 70 % der Theorie,
Schmelzpunkt: 187-189°C (Zers.)
$C_{21}H_{26}ClNO_5$ (407,90)

| | | | | |
|---|---|---|---|---|
| Ber.: | N | 3,43 | Cl | 8,69 |
| Gef.: | | 3,44 | | 8,66 |

## Beispiel M

**2-(2-Chlorbenzyl)-6,7-dimethoxy-3,4-dihydroisochinolinium-hydrogensulfat**

a) N-(2-Chlorbenzyl)-2-(3,4-dimethoxyphenyl)-äthylamin

80,3 g (0,443 Mol) 2-(3,4-Dimethoxyphenyl)-äthylamin und 62 g (0,443 Mol) o-Chlorbenzaldehyd werden in 500 ml Toluol am Wasserabscheider eine Stunde lang gekocht. Man dampft ein, nimmt den öligen Rückstand in 500 ml Methanol auf und versetzt unter starkem Kühlen und Rühren mit einer Lösung von 16,9 g (0,443 Mol) Natriumborhydrid in 50 ml Wasser. Nach beendeter Zugabe rührt man eine Stunde bei Zimmertemperatur nach, engt im Vakuum ein und destilliert den Rückstand im Vakuum.
Ausbeute: 108,8 g (80,2 % der Theorie),
$Kp_{16,9\ Pa}$: 188-190°C.

b) N-Formyl-N-(2-chlorbenzyl)-2-(3,4-dimethoxyphenyl)-äthylamin

88,4 g (0,29 Mol) N-(2-Chlorbenzyl)-2-(3,4-dimethoxyphenyl)-äthylamin werden mit 52,5 g (0,34 Mol) Chloralhydrat vermischt und 30 Minuten lang auf 120°C erhitzt. Nach dem Abkühlen versetzt man mit Essigester, wäscht mit Wasser, trocknet und dampft ein.
Ausbeute: 103 g (100 % der Theorie).

c) 2-(2-Chlorbenzyl)-6,7-dimethoxy-3,4-dihydroisochinolinium-hydrogensulfat

12,1 g N-Formyl-N-(2-chlorbenzyl)-2-(3,4-dimethoxyphenyl)-äthylamin werden in 50 ml Toluol gelöst, mit 15,2 g = 9 ml Phosphoroxychlorid versetzt und 1,5 Stunden am Rückfluß gekocht. Man dampft ein, versetzt den Rückstand mit Eis, stellt mit Kaliumcarbonat alkalisch und extrahiert mit Essigester. Der Essigesterextrakt wird eingedampft. Den erhaltenen Rückstand löst man in Isopropanol und versetzt mit 2 ml konz. Schwefelsäure. Man saugt nach dem Kühlen ab, wäscht mit Isopropanol und Äther und trocknet.

Ausbeute: 8,6 g (62,7 % der Theorie),

Schmelzpunkt: 226-228°C

$C_{18}H_{20}ClNO_6S$ (413,89)

| | | | | | | | | | | |
|---|---|---|---|---|---|---|---|---|---|---|
| Ber.: | C | 52,24 | H | 4,87 | N | 3,38 | Cl | 8,57 | S | 7,75 |
| Gef.: | | 52,44 | | 4,92 | | 3,35 | | 8,43 | | 7,98 |

Beispiel 1

Tabletten zu 300 mg 2-(2-Chlorbenzyl)-6,7-dimethoxy-3,4-dihydro-isochinolinium-hydrogensulfat

Zusammensetzung:

1 Tablette enthält:

| | |
|---|---|
| Wirksubstanz | 300,0 mg |
| Milchzucker | 120,0 mg |
| Cellulose mikrokristallin | 100,0 mg |
| Maisstärke | 72,0 mg |
| Polyvinylpyrrolidon | 6,0 mg |
| Magnesiumstearat | 2,0 mg |
| | 600,0 mg |

Herstellung:

Die Wirksubstanz wird mit Milchzucker, Cellulose und Maisstärke gemischt und mit einer 15%igen Lösung von Polyvinylpyrrolidon in Wasser granuliert. Die feuchte Masse wird durch ein Sieb geschlagen, auf Horden ausgebreitet und bei 45°C getrocknet. Nach einer nochmaligen Siebung wird Magnesiumstearat zugemischt und die Mischung zu Tabletten verpreßt.

| | |
|---|---|
| Tablettengewicht: | 600 mg |
| Stempel: | 13 mm Durchmesser, biplan beidseitige Facette und einseitige Teilkerbe |

Beispiel 2

Dragées zu 250 mg 2-(2-Chlorbenzyl)-6,7-dimethoxy-3,4-dihydro-isochinolinium-hydrogensulfat

1 Drageekern enthält:

| | |
|---|---|
| Wirksubstanz | 250,0 mg |

| | |
|---|---|
| Milchzucker | 100,0 mg |
| Cellulose mikrokristallin | 40,0 mg |
| Maisstärke | 84,0 mg |
| Polyvinylpyrrolidon | 5,0 mg |
| Magnesiumstearat | 1,0 mg |
| | 480,0 mg |

Herstellung:

Die Herstellung der preßfertigen Mischung erfolgt analog Beispiel 1.

Kerngewicht 480 mg

Stempel: 11 mm gewölbt mit einem Wölbungsradius von 10 mm.

Die Dragéekerne werden auf bekannte Art in einem Dragéekessel mit einer Zuckerschicht überzogen.

Dragéegewicht: 560 mg

Beispiel 3

Suppositorien zu 400 mg 2-(2-Chlorbenzyl)-6,7-dimethoxy-3,4-dihydro-isochinolinium-hydrogensulfat

1 Zäpfchen enthält:

| | |
|---|---|
| Wirksubstanz | 0,40 g |
| Zäpfchenmasse (z.B. Witepsol H 19 und Witepsol W 45) | 1,30 g |
| | 1,70 g |

Herstellung:

Das Hartfett wird geschmolzen. Bei 38°C wird die gemahlene Wirksubstanz in der Schmelze homogen dispergiert. Es wird auf 35°C abgekühlt und in schwach vorgekühlte Suppositorienformen ausgegossen.

Zäpfchengewicht: 1,7 g

Patentansprüche

1. Arzneimittel, enthaltend ein 3,4-Dihydro-isochinoliniumsalz der allgemeinen Formel

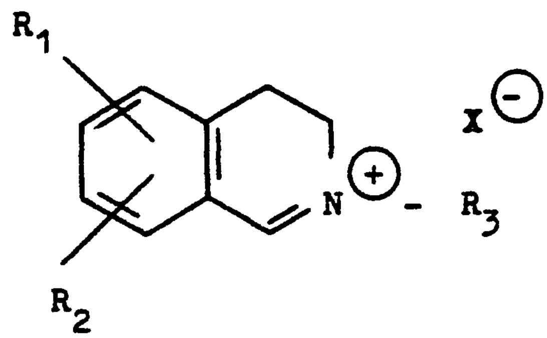

(I)

in der

$R_1$ und $R_2$, die gleich oder verschieden sein können, Wasserstoffatome, Alkyl- oder Alkoxygruppen mit jeweils 1 bis 3 Kohlenstoffatomen im Alkylteil,

$R_3$ eine Aralkylgruppe mit 7 bis 9 Kohlenstoffatomen, deren Phenylkern durch Halogenatome, Alkyl- oder Alkoxygruppen mit jeweils 1 bis 3 Kohlenstoffatomen im Alkylteil mono-, di- oder trisubstituiert sein kann, wobei die Substituenten des Phenylkerns gleich oder verschieden sein können, und

X das Anion einer physiologisch verträglichen anorganischen oder organischen Säure bedeuten, neben einem oder mehreren inerten Trägerstoffen und/oder Verdünnungsmitteln.

2. Arzneinittel gemäß Anspruch 1, enthaltend ein 3,4-Dihydro-isochinoliniumsalz der allgemeinen Formel I gemäß Anspruch 1, in der $R_1$ und $R_2$, die gleich oder verschieden sein können, Wasserstoffatome, Methyl- oder Methoxygruppen,

$R_3$ eine Benzyl-, Fluorbenzyl-, Chlorbenzyl-, Brombenzyl-, Methoxybenzyl- oder Trimethoxybenzylgruppe und
X das Anion einer physiologisch verträglichen anorganischen oder organischen Säure bedeuten, neben einem oder mehreren inerten Trägerstoffen und/oder Verdünnungsmitteln.

 

3. Arzneimittel gemäß Anspruch 1, enthaltend ein 3,4-Dihydro-isochinoliniumsalz der allgemeinen Formel I gemäß Anspruch 1, in der $R_1$ und $R_2$ je eine Methoxygruppe, $R_3$ eine 2-Chlorbenzyl-, 2-Methoxybenzyl- oder 3,4,5-Trimethoxybenzylgruppe und

X das Anion einer physiologisch verträglichen Säure bedeuten, neben einem oder mehreren inerten Trägerstoffen und/oder Verdünnungsmitteln.

4. Arzneimittel gemäß Anspruch 1, enthaltend 2-(2-Chlorbenzyl)-6,7-dimethoxy-3,4-dihydro-isochinoliniumhydrogensulfat neben einem oder mehreren inerten Trägerstoffen und/oder Verdünnungsmitteln.

5. Arzneimittel gemäß Anspruch 1, enthaltend 2-(2-Chlorbenzyl)-3,4-dihydro-isochinolinium-hydrogensulfat neben einem oder mehreren inerten Trägerstoffen und/oder Verdünnungsmitteln.

6. Arzneimittel gemäß Anspruch 1, enthaltend 2-(3,4,5-Trimethoxybenzyl)-6,7-dimethoxy-3,4-dihydroisochinolinium-hydrochlorid neben einem oder mehreren inerten Trägerstoffen und/oder Verdünnungsmitteln.

7. Arzneimittel gemäß den Ansprüchen 1 bis 6 zur Prophylaxe thromboembolischer Erkrankungen wie Coronarinfarkt, Cerebralinfarkt, sogn. transient ischaemic attacks, Amaurosis fugax sowie zur Prophylaxe der Arteriosklerose und der Metastasenbildung.

8. Verfahren zur Herstellung eines Arzneimittels gemäß den Ansprüchen 1 bis 7, dadurch gekennzeichnet, daß eine Verbindung gemäß den Ansprüchen 1 bis 6 in einen oder mehrere inerte Trägerstoffe und/oder Verdünnungsmittel eingearbeitet wird.

9. Verwendung einer Verbindung gemäß den Ansprüchen 1 bis 6 zur Prophylaxe thromboembolischer Erkrankungen wie Coronarinfarkt, Cerebralinfarkt, sogn. transient ischaemic attacks, Amaurosis fugax sowie zur Prophylaxe der Arteriosklerose und der Metastasenbildung.

10. 3,4-Dihydro-isochinoliniumsalze der allgemeinen Formel I gemäß Anspruch 1, in der
$R_1$ bis $R_3$ und X wie im Anspruch 1 definiert sind, als pharmazeutische Wirkstoffe.